(19) Europäisches Patentamt
European Patent Office
Office européen des brevets

(11) Veröffentlichungsnummer: **0 001 389**
**B1**

# (12) EUROPÄISCHE PATENTSCHRIFT

(45) Veröffentlichungstag der Patentschrift: **18.08.82**

(51) Int. Cl.³: **C 08 G 65/28,** C 08 G 18/48

(21) Anmeldenummer: **78100648.1**

(22) Anmeldetag: **11.08.78**

## (54) Verfahren zur Herstellung von Polyätherpolyolen und ihre Verwendung zur Herstellung von Polyurethanen.

(30) Priorität: **23.08.77 DE 2737951**

(43) Veröffentlichungstag der Anmeldung:
**18.04.79 Patentblatt 79/8**

(45) Bekanntmachung des Hinweises auf die Patenterteilung:
**18.08.82 Patentblatt 82/33**

(84) Benannte Vertragsstaaten:
**BE DE FR GB**

(56) Entgegenhaltungen:
**AT-B-262 620**
**DE-A-2 639 083**
**DE-A-2 721 186**

(73) Patentinhaber: **BAYER AG, Zentralbereich Patente, Marken und Lizenzen, D-5090 Leverkusen 1, Bayerwerk (DE)**

(72) Erfinder: **Wagner, Kuno, Dr., Am Kiesberg 8, D-5090 Leverkusen (DE)**

EP 0 001 389 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von Polyätherpolyolen und ihre Verwendung zur Herstellung von Polyurethanen

Die Erfindung betrifft ein Verfahren zur Herstellung neuartiger, mehrfunktioneller, gegebenenfalls weitgehend von Wasser-Alkoxylierungsprodukten freier, vergleichsweise niederviskoser, wenig gefärbter Polyalkylenglykoläther auf der Basis eines durch Selbstkondensation von Formaldehydhydrat unter saurer Katalyse hergestellten Gemisches mehrwertiger Alkohole, Hydroxyaldehyde und Hydroxyketone; die neuen Polyätherpolyole sind wertvolle Ausgangsmaterialien zur Herstellung von Polyurethanschaumstoffen.

Verfahren zur Herstellung von Polyalkylenglykoläthern sind an sich bekannt. Polyäther werden nach den Verfahren des Standes der Technik durch Polymerisation von Epoxiden mit sich selbst oder durch Anlagerung dieser Epoxide an Startkomponenten mit reaktionsfähigen Wasserstoffatomen hergestellt. Bevorzugte Startkomponenten bei den Verfahren des Standes der Technik sind beispielsweise Saccharose (DAS 1 064 938, DAS 1 176 358, DOS 1 443 022), Sorbit (GB 876 496, belgische Patentschrift 582 076 und Modern Plastics, Mai 1959, S. 151–154) sowie verschiedene di- und trifunktionelle Polyalkohole, wie z.B. Äthylenglykol, Propylenglykol, Trimethylolpropan oder Glycerin.

Polyätherpolyole mit einer Hydroxylfunktionalität von 8 bzw. 6 werden durch Umsetzung von Saccharose bzw. Sorbit (oder anderen sechswertigen Zuckeralkoholen) erhalten. Diese hochfunktionellen Polyäther eignen sich, wenn sie relativ niedrige Molekulargewichte aufweisen, besonders zur Herstellung von harten und halbharten Polyurethanschaumstoffen, die sich durch gute Dimensionsstabilität auszeichnen.

Für die Umsetzung von Saccharose und Sorbit mit Alkylenoxiden im technischen Massstab gilt als wesentliche Voraussetzung, dass das Reaktionsgemisch einwandfrei gerührt werden kann. Die bei der Umsetzung von Alkylenoxiden mit Hydroxylverbindungen auftretende hohe Wärmetönung lässt sich in ausreichendem Masse nur abführen, wenn das Reaktionsgemisch mit hoher Turbulenz gerührt werden kann.

Unter den Bedingungen der technischen Polyätherherstellung, das sind Temperaturen von 95–115° C und 0,5–3,5 atü Druck, sind indessen Mischungen von Alkylenoxiden mit Saccharose bzw. Sorbit nur schlecht rührbar. Das Problem der Rührbarkeit tritt vor allem bei Saccharose und zu Beginn der Alkylenoxidzugabe auf, wenn noch grosse Mengen an nicht umgesetztem festem Starter vorhanden sind. Schlecht rührbare Mischungen von Saccharose und Alkalihydroxid – das meist als Katalysator bei der Polyätherherstellung eingesetzt wird – können zur Karamelisierung bzw. zu Verkohlungsreaktionen an den beim Erhitzen des Reaktionsgemisches notwendigerweise heissen Wänden des Reaktionsgefässes führen. Mischungen von Sorbit und Alkylenoxiden sind bei Anwesenheit grosser Mengen nicht umgesetzten Sorbits ebenfalls nur sehr schlecht rührbar, da Sorbit noch als Festkörper vorliegt oder gerade bei den Reaktionstemperaturen zu schmelzen beginnnt (Fp.: 97,7° C) und die erhaltenen Schmelzen relativ hochviskos sind.

Überhitzungen bei Sorbitschmelzen, die in schlecht gerührten Reaktionsmischungen leicht auftreten können, können in Gegenwart von Alkalihydroxiden zur Bildung von sogenannten Sorbitanhydriden, den Sorbitanen, führen, was zu einem Verlust an Funktionalität bei den resultierenden Polyäthern und daher zu einer Verschlechterung der Eigenschaften der daraus hergestellten harten Polyurethanschaumstoffe führt.

Um diese Nachteile zu vermeiden, wurde schon vorgeschlagen, als Startkomponenten Mischungen von Saccharose oder Sorbit mit niederviskosen bi- oder trifunktionellen Polyalkoholen einzusetzen (DAS 1 285 741, DOS 1 443 372, DOS 2 241 242, DOS 2 521 739 und DOS 2 549 449) oder wässrige Lösungen der höherfunktionellen Starter zu verwenden.

Bei der Umsetzung von Saccharose bzw. Sorbit mit Alkylenoxid in wässriger Lösung oder in Mischung mit Glykolen finden jedoch leicht unerwünschte Nebenreaktionen statt, beispielsweise die teilweise Hydrolyse des Alkylenoxids durch das als Reaktionsmedium verwendete Wasser. Das hydrolysierte Alkylenoxid, die daraus durch Reaktion mit weiterem Alkylenoxid gebildeten Polyalkylenglykole und die anderen entstandenen Nebenprodukte, die durch eine starke Dunkelfärbung des Reaktionsgemischs angezeigt werden, wirken sich nachteilig auf die Eigenschaften der aus solchen Saccharose – bzw. Sorbithydroxyalkyläthern hergestellten harten bzw. halbharten Polyurethanschäume aus.

Ein Nachteil der aus derart hergestellten Saccharosepolyäthern erhaltenen harten Polyurethanschaumstoffe ist ihr oft geringer Anteil an geschlossenen Zellen und ihr hiermit im Zusammenhang stehendes schlechtes Wärmeisolationsvermögen.

Der hohe Anteil an bifunktionellen und trifunktionellen Nebenprodukten bei derartigen Polyäthern führt ausserdem dazu, dass die aus diesen Polyäthergemischen hergestellten harten Polyurethanschaumstoffe nur eine verminderte Dimensionsstabilität aufweisen.

Polyätherpolyole, die durch Umsetzung von Saccharose oder Saccharose/Glykol-Mischungen hergestellt wurden und mittlere Molekulargewichte von 500 bis 1500 aufweisen, sind relativ hochviskose Flüssigkeiten. Aufgrund der hohen Viskosität kommt es beim Schäumvorgang zu einem verminderten Fliessvermögen der fertig formulierten Reaktionsmischung, was zu einer verschlechterten Formausfüllung beim Formschäumen führt. Ausserdem bildet sich auch eine ungleichmässige Rohdichteverteilung innerhalb des Polyurethanschaums aus, was eine Verminderung der Druckfestigkeitswerte bewirkt.

In der österreichischen Patentschrift 262 620 wird die Epoxidierung reduzierender Mono- oder Polysaccharide unter (saurer) Bortrihalogenid-Katalyse in Gegenwart von höchstens 15 Gew.-% Wasser, mehrwertigen Alkoholen oder deren Äther oder Ester beschrieben.

Die kristallinen Zucker (Mono-/Oligosaccharide) sind ohne solche Lösungsmittel praktisch nicht drucklos propoxylierbar. Andererseits senkt der Zusatz von z.B. Wasser (gleichbedeutend mit einem Glykol, das aus $H_2O$ plus 1,2-Epoxid entsteht) in der notwendigen relativ hohen Menge erheblich die mittlere Funktionalität des entstehenden Polyäthers. Trotzdem ist die Viskosität der Zuckeräther jeweils höher als die der erfindungsgemässen Formosepolyäther mit gleich hohem Wasserzusatz.

Während die Mono- und Oligosaccharide naturgemäss bestimmte Funktionalitätsniveaus aufweisen, lassen sich erfindungsgemäss Formosen in beliebigen mittleren Funktionalitäten herstellen und auch praktisch wasserfrei problemlos propoxylieren.

Polyäther, die sich für die Herstellung von flexiblen Polyurethanschaumstoffen eignen, werden meist nach an sich bekannten Verfahren durch Umsetzung von trifunktionellen Polyolen, wie Glycerin oder Trimethylolpropan, mit Propylenoxid oder Äthylenoxid oder einem Gemisch aus Propylenoxid und Äthylenoxid erhalten. Häufig wird auch die Starterkomponente zunächst mit Propylenoxid und anschliessend mit Äthylenoxid umgesetzt, so dass Polyäther mit vorwiegend primären endständigen Hydroxylgruppen entstehen.

Polyurethanschaumstoffe, die aus derartigen Polyätherpolyolen hergestellt wurden, werden allerdings häufig den an sie gestellten Anforderungen bezüglich der Stauchhärte nicht voll gerechnet. Um zu flexiblen Polyurethanschaumstoffen mit erhöhter Stauchhärte zu gelangen, wurde daher schon vorgeschlagen, bi- und trifunktionelle Starter mit Sorbit oder Saccharose zu mischen und diese Mischungen mit einem grossen Überschuss an Äthylenoxid zu Polyätherpolyolen mit einem mittleren Molekulargewicht von 1000–10 000 umzusetzen (DOS 2 521 739 und DOS 2 549 449). Auch die Umsetzung von Sorbit allein mit Alkylenoxiden zu relativ hochmolekularen Polyätherpolyolen mit einer Hydroxylzahl von 20–60 ist bekannt.

Bei der Herstellung derartiger Polyätherpolyole nach den Verfahren des Standes der Technik treten jedoch ebenfalls Schwierigkeiten dadurch auf, dass die Mischungen der Startkomponenten bei Raumtemperatur oder nur wenig erhöhter Temperatur entweder breiige Konsistenz haben oder Flüssigkeiten mit einer relativ hohen Viskosität sind. Starterkomponenten dieser Art können daher nicht ohne weiteres durch Rohrleitungen gefördert werden und erfordern aus diesem Grund aufwendige Einrichtungen, wenn die Polyätherpolyole im grosstechnischen Massstab hergestellt werden.

Auch die einwandfreie Rührbarkeit dieser Mischungen mit hoher Turbulenz ist – wie bei den Hartschaumpolyäthern – nicht ohne weiteres möglich. Aus diesem Grund ist die Reaktionsgeschwindigkeit der Alkylenoxide vermindert, was zu geringen Raum-Zeit-Ausbeuten bei der Herstellung der Polyätherpolyole führt. Nebenprodukte, die sich durch Zersetzung der schlecht gerührten Reaktionsmischungen an den heissen Wänden des Reaktionsgefässes bilden, führen ausserdem zu Qualitätsverschlechterungen der resultierenden Polyätherpolyole hinsichtlich der Hydroxylfunktionalität; häufig werden gelb bis braun gefärbte Polyäther erhalten.

Es besteht daher der Bedarf nach einem Verfahren zur Herstellung von Polyalkylenglykoläthern, welches unter weitgehender Vermeidung der Nachteile der geschilderten Verfahren des Standes der Technik auf einfache Weise Polyätherpolyole ohne die erwähnten nachteiligen Eigenschaften und mit verschiedener, je nach Anwendungszweck einstellbarer Funktionalität zugänglich macht.

Gemäss einem eigenen älteren Vorschlag (deutsche Patentanmeldung P 26 39 083.0) werden Polyätherpolyole mit einem durchschnittlichen Molekulargewicht von 200 bis 10 000 und mit einer durchschnittlichen Hydroxylfunktionalität von 2,0 bis 7,0 hergestellt, indem man ein oder mehrere Alkylenoxide, gegebenenfalls nacheinander, mit einem durch Selbstkondensation von Formaldehydhydrat und anschliessende Reduktion der Kondensationsprodukte hergestellten Gemisch mehrwertiger Alkohole, welches gegebenenfalls mit zusätzlichen zweiwertigen und/oder dreiwertigen Alkoholen und/oder Mono- oder Polyaminen abgemischt wurde, zur Reaktion gebracht werden (im folgenden sollen die durch Selbstkondensation von Formaldehydrat hergestellten Gemische von mehrwertigen Alkoholen, Hydroxyaldehyden und Hydroxyketonen als «Formose» und das daraus durch Hydrierung hergestellte Polyolgemisch als «Formit» bezeichnet werden).

In weiterer Ausbildung des Verfahrens der erwähnten älteren Anmeldung wurde nunmehr überraschenderweise gefunden, dass Polyätherpolyole mit hervorragenden Eigenschaften in einfacher, wirtschaftlicher und reproduzierbarer Weise direkt durch Alkoxylierung von Formose in Gegenwart stark saurer Katalysatoren hergestellt werden können.

Gegenstand der vorliegenden Erfindung ist somit ein Verfahren zur Herstellung von Polyätherpolyolen mit einem durchschnittlichen Molekulargewicht im Bereich von 200 bis 10 000 und mit einer durchschnittlichen Hydroxylfunktionalität von 2,0 bis 7,0 durch Anlagerung eines oder mehrerer cyclischer Äther, in Gegenwart eines sauren Katalysators, an einen Starter, bestehend aus Zuckern und gegebenenfalls mehrwertigen Alkoholen, dadurch gekennzeichnet, dass als Starter

A) Formose, welche gegebenenfalls in α-Stellung aldolisiert ist, oder

B) flüssige Gemische aus

a) hoch- und/oder niedermolekularen Polyhydroxylverbindungen und/oder Mono- oder Disacchariden und/oder natürlichen oder künstlichen Invertzuckern und

b) Formose, welche gegebenenfalls in α-Stellung aldolisiert ist,

eingesetzt werden, wobei Formosen ausgenommen sind, welche direkt aus formaldehydhaltigen Synthesegasen hergestellt worden sind.

Es ist als überraschend anzusehen, dass nach dem erfindungsgemässen Verfahren in reproduzierbarer Weise qualitativ hochwertige, nicht oder nur schwach gefärbte Produkte ohne störende Nebenreaktionen (beispielsweise Dehydratisierung der Zucker unter Dunkelfärbung) erhalten werden. Versucht man beispielsweise, Formose durch basisch katalysierte ringöffnende Polyadditionsreaktionen mit Oxiranen zu alkoxylieren, so laufen zahlreiche – noch weitgehend unbekannte – Nebenreaktionen unter starker Schwarzfärbung des Reaktionsgemisches ab, so dass eine Herstellung standardisierter Polyäther auf Basis von Formose auf diesem Wege nicht möglich ist. Andererseits gelingt es aber auch nicht, die konventionellen Starter für Polyäther auf Zuckerbasis (beispielsweise Glucose oder Rohrzucker) mittels saurer Katalysatoren zu alkoxylieren.

Überraschenderweise laufen beim erfindungsgemässen Verfahren neben der Addition der cyclischen Äther an die Hydroxylgruppen der Formose in hoher Ausbeute auch Acetalisierungs- bzw. Ketalisierungsreaktionen an den Carbonylfunktionen der Formose ab. Dies hat den Vorteil, dass bei der Verwendung der erfindungsgemäss erhaltenen Polyätherpolyole zur Herstellung von Polyurethanschaumstoffen keine Karamelisierungsreaktionen unter unangenehmer Geruchsbildung und eine Kernverfärbung der Schaumstoffe wie bei den herkömmlichen Zuckerpolyäthern auftreten.

Im erfindungsgemässen Verfahren können Formosen beliebiger Art eingesetzt werden. Die Herstellung von Gemischen mehrwertiger Alkohole, Hydroxyaldehyde und Hydroxyketone durch Selbstkondensation des Formaldehydhydrats wird in zahlreichen Literaturstellen beschrieben. Beispielsweise seien in diesem Zusammenhang Butlerow und Loew, Annalen *120*, 295 (1981), bzw. J. pr. Chem. *33*, 321 (1886), Pfeil, Chemische Berichte *84*, 229 (1951), Pfeil und Schroth, Chemische Berichte *85*, 303 (1952), R.D. Partridge und A.H. Weiss, Carbohydrate Research *24*, 29–44 (1972), die Formosen aus Glycerinaldehyd bzw. Dioxyaceton nach Emil Fischer, die deutschen Patentschriften 822 385, 330 951 und 884 794 sowie die US-Patentschriften 2 224 910, 2 269 935 und 2 272 378 und die englische Patentschrift 513 708 genannt. Diese bekannten Verfahren des Standes der Technik sind jedoch mit einer Reihe von Nachteilen behaftet (toxikologisch bedenkliche Katalysatoren, schlechte Raum-Zeit-Ausbeuten, gefärbte Nebenprodukte); erfindungsgemäss setzt man daher vorzugsweise Formosen als Starter ein, welche gemäss neuen Verfahren der Anmelderin hergestellt wurden.

Eines dieser neuen Verfahren besteht darin, dass man die Kondensation des Formaldehydhydrats in Gegenwart von löslichen oder unlöslichen Blei(II)-salzen, gegebenenfalls gebunden an hochmolekulare Träger, als Katalysator und eines Gemisches aus Hydroxyaldehyden und Hydroxyketonen als Cokatalysator ablaufen lässt, wie es bei der Kondensation von Formaldehydhydrat entsteht und welches durch folgende Molverhältnisse charakterisiert ist:

Verbindungen mit 3 C-Atomen/Verbindungen mit 4 C-Atomen: 0,5–2,0

Verbindungen mit 4 C-Atomen/Verbindungen mit 5 C-Atomen: 0,2–2,0

Verbindungen mit 5 C-Atomen/Verbindungen mit 6 C-Atomen: 0,5–5,0

wobei der Anteil der Komponenten mit 3 bis 6 C-Atomen mindestens 75 Gew.-%, vorzugsweise mehr als 85 Gew.-%, bezogen auf gesamten Cokatalysator, beträgt.

Die Reaktionstemperatur liegt dabei im allgemeinen zwischen 70 und 110° C, bevorzugt zwischen 80 und 100° C und der pH-Wert der Reaktionslösung wird durch kontrollierte Zugabe einer anorganischen oder organischen Base bis zu einem Umsatz von 10–60%, vorzugsweise 30–50%, auf einen Wert von 6,0–8,0, bevorzugt 6,5–7,0, und anschliessend auf einen Wert von 4,0–6,0, bevorzugt 5,0–6,0, eingestellt. Überraschenderweise wurde gefunden, dass sich die Produktverteilung der entsprechenden Polyol-, Hydroxyaldehyd- und Hydroxyketongemische durch diese spezielle pH-Führung und durch anschliessende Kühlung bei verschiedenen hohen Restformaldehydgehalten (0 bis 10 Gew.-%, vorzugsweise 0,5 bis 6 Gew.-%) in reproduzierbarer Weise variieren lässt.

Nachdem man die Selbstkondensation des Formaldehydhydrats durch Kühlen und/oder durch Desaktivierung des bleihaltigen Katalysators mittels Säuren unterbrochen hat, wird der Katalysator in an sich bekannter Weise entfernt und das in den Produkten enthaltene Wasser verdampft. Hinsichtlich näherer Einzelheiten sei auf die deutsche Offenlegungsschrift 2 639 084 verwiesen.

Eine weitere Möglichkeit, in hoher Raum-Zeit-Ausbeute hochkonzentrierte farblose Formosen herzustellen, besteht darin, wässrige Formaldehydlösungen und/oder Paraformaldehyd-Dispersionen in Gegenwart eines löslichen oder unlöslichen Metallkatalysators und eines durch teilweise Oxidation eines zwei- oder mehrwertigen, mindestens zwei benachbarte Hydroxygruppen aufweisenden Alkohols mit einem Molekulargewicht zwischen 62 und 242 bzw. eines Gemisches derartiger Alkohole dargestellten Cokatalysators zu kondensieren, wobei man den pH-Wert der Reaktionslösung durch gesteuerte Zufuhr einer Base bis zu einem Umsatz von 5 bis 40% zwischen 6,0 und 9,0 zu halten und anschliessend bis zum Abbruch der Kondensationsreaktion auf 4,5 bis 8,0 einzustellen, so dass er nun um 1,0 bis 2,0 Einheiten tiefer liegt als in der ersten Reaktionsphase, dann die Reaktion bei einem Restgehalt von 0 bis 10 Gew.-% Formaldehyd durch Desaktivierung des Katalysators unterbricht und den Katalysator entfernt. Im Detail wird diese Arbeitsweise in der DOS 2 714 084 beschrieben.

Für das erfindungsgemässe Verfahren können selbstverständlich auch Formosen eingesetzt werden, welche durch Kondensation von Formaldehyd in Gegenwart eines Metallkatalysators und mehr als 10 Gew.-%, bezogen auf Formaldehyd, eines oder mehrerer zwei- oder mehrwertiger niedermolekularer Alkohole und/oder höhermolekularer Polyhydroxylverbindungen hergestellt wurden. Derartige Formose-Polyolgemische sind Gegenstand von DOS 2 714 104.

Von dem erfindungsgemässen Verfahren sind Formosen, die direkt aus formaldehydhaltigen Synthesegasen, wie sie bei der grosstechnischen Herstellung von Formaldehyd anfallen, hergestellt werden, ausgenommen.

Selbstverständlich können erfindungsgemäss auch Formosen eingesetzt werden, welche durch überschüssigen Formaldehyd in ihre Halbacetale übergeführt oder durch Reaktion mit Formaldehyd in Gegenwart von Basen α-methyloliert wurden.

Je nach der Reaktionsführung bei der Formaldehydkondensation lassen sich die Eigenschaften der Formose (mittlere Hydroxylfunktionalität; Verzweigungsgrad; Gehalt an reduzierenden Gruppen) in weiten Grenzen variieren. Im allgemeinen ist das mittlere Molekulargewicht und damit die Hydroxylfunktionalität der Formosen um so höher, je weiter die Kondensationsreaktion geführt wird, d.h. je weniger Restformaldehyd beim Abbruch der Kondensationsreaktion vorhanden ist. So wird etwa, wenn die Kondensationsreaktion bis zu einem Restformaldehydgehalt von 0 bis 1,5 Gew.-% geführt wird, eine Formose erhalten, welche ca. 25 Gew.-% an Anteilen mit 5 C-Atomen, 45 Gew.-% an Verbindungen mit 6 C-Atomen und ca. 20 Gew.-% an Verbindungen mit 7 und mehr C-Atomen enthält. Dagegen werden zusammen nur ca. 10% an Polyolen, Hydroxyketonen und Hydroxyaldehyden mit 2,3 und 4 C-Atomen erhalten. Dies entspricht einer mittleren Hydroxylfunktionalität von ca. 5. Polyäther, die erfindungsgemäss durch Alkoxylierung eines derartigen Startergemisches hergestellt wurden, eignen sich vorzüglich zur Herstellung harter Polyurethanschaumstoffe.

Durch Abbruch der Formaldehydselbstkondensation bei etwas höheren Restformaldehydgehalten werden aber, wie oben erläutert, auch noch andere Komponentenverteilungen der Startergemische erhalten. So ergibt sich bei einem Abbruch der Kondensationsreaktion bei 2 bis 2,5% Formaldehydgehalt ein Gemisch mehrwertiger Alkohole, Hydroxyaldehyde und Hydroxyketone mit einer mittleren Hydroxylfunktionalität von ca. 4. Durch Propoxylierung wird daraus ein Polyäther erhalten, der sich ebenfalls für die Herstellung harter Polyurethanschaumstoffe vorzüglich eignet.

Noch andere Komponentenverteilungen mit niedrigerer durchschnittlicher Hydroxylfunktionalität werden erhalten, wenn man die Kondsensationsreaktion bei Restformaldehydgehalten abbricht, die noch höher liegen als 2,5. Diese Startergemische mit niedriger Hydroxylfunktionalität lassen sich zu Polyäthern umsetzen, die sich für die Herstellung flexibler Polyurethanschaumstoffe eignen. Ihre Viskosität ist niedriger als jene von handelsüblichen Polyäthern auf Trimethylolpropan- oder Glycerinbasis mit gleicher Funktionalität, was zu einem verbesserten Werteniveau der daraus hergestellten Polyurethanschäume führt. Auf Grund der geringeren Viskosität ist das Fliessverhalten der fertig formulierten Reaktionsmischung deutlich verbessert, was beispielsweise zu einem gleichmässigeren Ausfüllen der Schäumform führt, wenn die Polyäther zur Herstellung von geschäumten Formteilen Verwendung finden sollen.

Erfindungsgemäss werden bevorzugt Formosen mit einem durchschnittlichen Molekulargewicht zwischen 92 und 360, besonders bevorzugt zwischen 100 und 240, und einem Gehalt an reduzierenden Verbindungen (berechnet als Glucose vom Molekulargewicht 180) von 4 bis 85 Gew.-%, besonders bevorzugt 6 bis 72 Gew.-%, eingesetzt.

Durch Abmischen der Formose mit di- oder höherfunktionellen niedermolekularen Alkoholen lässt sich gegebenenfalls die Funktionalität des Startergemisches in gewünschter Weise weiter variieren, wenn bestimmte anwendungstechnische Effekte der resultierenden Polyäther erreicht werden sollen. Als derartige niedermolekulare mehrwertige Alkohole (Molekulargewicht bis ca. 300) kommen beispielsweise Äthylenglykol, Propandiol-1,2, Propandiol-1,3, Butandiol-1,4, Diäthylenglykol, Dipropylenglykol, Triäthylenglykol, Tetraäthylenglykol, Dibutylenglykol, Glycerin, Trimethylolpropan, Pentaerythrit, Sorbit, Butantriole und Hexantriole sowie Oxäthylierungsprodukte dieser Alkohole bzw. auch hydrierte Formose (Formit) in Frage.

Überraschenderweise ist Formose in der Lage, relativ hohe Mengen an kristallisierten Mono- und Disacchariden wie beispielsweise Glucose, Maltose oder Rohrzucker zu lösen, ebenso natürliche Invertzucker (beispielsweise Bienenhonig) oder künstliche Invertzucker, z.B. Hydrolysate von Rohrzucker, ferner Hydrolysate von Mais- und Kartoffelstärke und von Pektinstoffen (Amylose und Amylopektine), ebenso Hydrolysate von beliebigen anderen Di- und/oder Polysacchariden, z.B. Trehalose, Galactose, Raffinose, Cellulose und Dextrinen. Dies ist technisch von besonderem Interesse, weil derartige kristallisierte Mono- bzw. Disaccharide sich in reiner Form nicht analog zum erfindungsgemässen Verfahren alkoxylieren lassen. Bevorzugt setzt man derartige Mono- und Disaccharide im erfindungsgemässen Verfahren in Form einer 20 bis 80 Gew.-%igen, besonders bevorzugt 30 bis 70 Gew.-%igen Lösung in Formose ein. Solche Lösungen lassen sich sehr einfach herstellen, indem man wässrige Zuckerlösungen mit Formose mischt und die Lösungen, beispielsweise im Dünnschichtverdampfer, auf Wassergehalte von ca. 0,5 bis 5 Gew.-%, bevorzugt 0,7 bis 3,5 Gew.-%, einengt.

Für das erfindungsgemässe Verfahren kommen alle sauren Katalysatoren in Betracht, wie sie für Friedel-Crafts-Reaktionen bzw. für kationisch initiierte Polymerisationen bekannt sind. Es sind dies einerseits starke anorganische oder organische Säuren wie Schwefelsäure, Perchlorsäure, Chlorsulfonsäure, Fluorsulfonsäure, aliphatische und aromatische Sulfonsäuren wie Methansulfonsäure, Butansulfonsäure, Trifluormethansulfonsäure, Perfluoralkancarbonsäuren, Benzolsulfonsäure und p-Toluolsulfonsäure, Lewis-Säuren wie z.B. Eisen-III-chlorid, Eisen-III-bromid, Antimon-V-chlorid, Aluminiumtrichlorid, Titantetrachlorid und Zinntetrachlorid bzw. die entsprechenden Fluoride, Bortrichlorid, Bortrifluorid sowie Anlagerungsverbindungen der Borhalogenide an Äther, Carbonsäuren, Carbonsäureanhydride, Carbonsäureester, Amine, Nitrile und Mono- oder Dicarbonsäureamide, z.B. die Addukte an Diäthyläther, Tetrahydrofuran, Di- n- Butyläther, Anisol, Essigester, Acetanhydrid, Acetonitril, Dimethylformamid, Eisessig oder Wasser. Oxoniumsalze und Carboxoniumsalze von Borhalogeniden wie Triäthyloxoniumfluoroborat und 2-Methyl-dioxolenium-fluoroborat und Fluoroborate von Aryldiazoniumverbindungen, welche bei erhöhter Temperatur unter Stickstoffabspaltung in Arylkationen übergehen wie p-Nitrophenyl-diazonium-fluoroborat sind gleichfalls erfindungsgemäss geeignete Katalysatoren. Bezüglich weiterer für kationische Polymerisationen gebräuchlicher Katalysatoren und Katalysatorsysteme sei auf die deutschen Patentschriften 741 478 und 766 208 sowie die französische Patentschrift 898 269 hingewiesen. Erfindungsgemäss bevorzugter Katalysator sind Bortrifluorid bzw. Addukte von Bortrifluorid an Säuren, Anhydride, gemischte Anhydride und cyclische Anhydride; besonders bevorzugt sind die Addukte von Bortrifluorid an Essigsäure, Propionsäure und Essigsäureanhydrid. Die Katalysatoren werden im erfindungsgemässen Verfahren im allgemeinen in einer Menge von 0,001 bis 5 Gew.-%, bevorzugt 0,05 bis 2 Gew.-%, besonders bevorzugt 0,2 bis 1 Gew.-% (bezogen auf gesamtes Reaktionsgemisch) eingesetzt.

Bekanntlich wird die ringöffnende kationische Polyaddition von cyclischen Äthern durch Verbindungen mit aktiven Wasserstoffatomen, insbesondere Hydroxylverbindungen und Wasser, stark beschleunigt. Als derartige Cokatalysatoren kommen erfindungsgemäss einerseits die bereits oben als Abmischkomponente genannten 2- und mehrwertigen Alkohole mit einem Molekulargewicht von 62 bis ca. 300 in Frage, andererseits an sich bekannte, Hydroxylgruppen aufweisende lineare oder verzweigte Polyäther, Polyester, Polyacetale und Polycarbondate mit einem Molekulargewicht zwischen ca. 300 und 4 000, wie sie weiter unten als gegebenenfalls mitzuverwendende Ausgangskomponenten bei der Herstellung von Polyurethankunststoffen beschrieben werden.

Auch Umsetzungsprodukte von Polyolen mit cyclischen Säureanhydriden, die zu Polyäthern mit freien Carboxylgruppen führen, und durch Urethangruppen modifizierte Verbindungen, wie sie bei der Umsetzung der obengenannten Hydroxylverbindungen mit einer weniger als äquivalenten Menge an Polyisocyanat erhalten werden, sind als Cokatalysatoren für das erfindungsgemässe Verfahren geeignet. Alle diese cocatalytisch wirkenden, Hydroxylgruppen aufweisenden Verbindungen können im erfindungsgemässen Verfahren an sich in beliebigen Mengen, vorzugsweise jedoch in Anteilen von 2–100 Gew.-% (bezogen auf Formose) mitverwendet werden).

Erfindungsgemäss besonders bevorzugter Cokatalysator ist Wasser. Wird Wasser als Cokatalysator eingesetzt, dann sollte im allgemeinen eine Menge von maximal 4 Gew.-%, bezogen auf Startergemisch, nicht überschritten werden. Nur wenn die erfindungsgemäss als Katalysator besonders bevorzugten Addukte von Bortrifluorid an Carbonsäuren bzw. Carbonsäureanhydride eingesetzt werden, dann lassen sich auch grössere Mengen an Wasser (0,5 bis ca. 20 Gew.-%, bevorzugt 0,5 bis 10 Gew.-%) an Wasser mitverwenden.

Als cyclische Äther kommen beim erfindungsgemässen Verfahren insbesondere Epoxide mit 2 bis 8 Kohlenstoffatomen wie beispielsweise Äthylenoxid, Propylenoxid, 1,2-Butylenoxid, 2,3-Butylenoxid, Styroloxid, Cyclopentenoxid oder Cyclohexenoxid in Frage, aber auch cyclische Äther mit 3 bis 5 C-Atomen im Ring wie z.B. Trimethylenoxid, Tetramethylenoxyd (Tetrahydrofuran) oder Pentamethylenoxyd (Tetrahydropyran). Auch halogensubstituierte bzw. olefinische Doppelbindungen aufweisende Alkylenoxide wie z.B. Epichlorhydrin, Vinyloxiran, Vinylcyclohexenoxid oder Methacrylsäureglycidester können eingesetzt werden.

Die erfindungsgemässe Alkoxylierungsreaktion kann sowohl unter ausschliesslicher Verwendung eines einzigen der genannten Alkylenoxide als auch unter Verwendung von beliebigen Gemischen der Alkylenoxide durchgeführt werden. Es ist auch möglich, beim erfindungsgemässen Verfahren verschiedene Alkylenoxide nacheinander in einem Reaktionsansatz zur Herstellung von sogenannten Blockcopolyäthern einzusetzen. Vorzugsweise werden erfindungsgemäss Äthylenoxid und/oder Propylenoxid verwendet.

Die Reaktionstemperaturen können beim erfindungsgemässen Verfahren in weiten Grenzen variiert werden. Im allgemeinen arbeitet man zwischen 5 und 120° C, vorzugsweise zwischen 20 und 90° C, besonders bevorzugt zwischen 30 und 70° C. Die Umsetzung mit den oben beschriebenen cyclischen Äthern kann sowohl bei erhöhtem Druck als auch bei Normaldruck bzw. leichtem Unterdruck erfolgen. Vorzugsweise werden Drucke zwischen 0,5 und 5 bar, besonders bevorzugt zwischen 1 und 3 bar, angewandt. Vorzugsweise wird das erfindungsgemässe Verfahren in einer Inertgasatmosphäre (Stickstoff oder Edelgase) ausgeführt.

Gegebenenfalls kann das erfindungsgemässe Verfahren auch in Gegenwart eines inerten Lösungsmittels wie z.B. Toluol, Xylol oder Perchloräthylen ausgeführt werden. Vorzugsweise wird

jedoch in Abwesenheit von Lösungsmitteln gearbeitet.

Im allgemeinen legt man erfindungsgemäss die Formose (gegebenenfalls im Gemisch mit Zukkern und/oder Polyhydroxylverbindungen und/oder Wasser) in einem geeigneten Rührkessel vor, spült mehrmals mit Stickstoff und setzt den Katalysator unter Rühren zu (sollen erfindungsgemäss Polyäther mit hohem Molekulargewicht hergestellt werden, also relativ hohe Mengen an cyclischen Äthern an das Startergemisch angelagert werden, dann ist es zweckmässig, zunächst nur einen Teil des Katalysators vorzulegen und die Restmenge des Katalysators portionsweise parallel zur Zugabe des cyclischen Äthers zuzusetzen). Danach wird der cyclische Äther portionsweise in das Startergemisch eingerührt. Nach Beendigung der Polyadditionsreaktion wird der Ansatz evakuiert, um Spuren von restlichem Monomeren zu entfernen, neutralisiert und von unlöslichen Metallsalzen durch Filtration getrennt.

Die erfindungsgemäss hergestellten Polyäther sind klare, farblose bis gelbliche Flüssigkeiten, deren Viskosität je nach Hydroxylzahl und Funktionalität zwischen 400 mPa.s/25° C (bei Polyäthern mit einer Funktionalität von 3 und OH-Zahlen von 60 bis 55) und ca. 30 000 mPa.s/25° C (z.B. bei Polyäthern der Funktionalität 4,6 und einer OH-Zahl von 556) schwankt. Die Viskosität der erhaltenen Polyäther sind gegenüber handelsüblichen Polyäthern bei vergleichbarer Funktionalität und Hydroxylzahl deutlich erniedrigt. Durch Variation der Hydroxylzahl und der Komponentenverteilung der Startergemische, sowie der gegebenenfalls zugesetzten Diole und/oder Triole können Produkte hergestellt werden, deren Viskosität dem jeweiligen Einsatzzweck optimal angepasst ist.

Die erfindungsgemäss hergestellten Polyätherpolyole können – gegebenenfalls zusammen mit weiteren an sich bekannten höhermolekularen Verbindungen, welche gegenüber Isocyanaten reaktive Gruppen aufweisen, und/oder Kettenverlängerungsmitteln – mit Polyisocyananten zu homogenen oder zellulären Polyurethankunststoffen umgesetzt werden.

Für die Herstellung von Polyurethanhartschaumstoffen kommen insbesondere Formose-Polyäther mit OH-Zahlen von ca. 300 bis 650 in Frage, während erfindungsgemäss hergestellte Polyäther mit OH-Zahlen von ca. 25 bis 60, vorzugsweise für die Herstellung von weichen Schaumstoffen, eingesetzt werden. Die erfindungsgemässen Polyätherpolyole sind darüber hinaus auch wertvolle Vorprodukte für die Herstellung von Emulgatoren und Tensiden; sie können auch als Verdickungsmittel für Farbpigmentpasten eingesetzt werden.

Gegenstand der vorliegenden Erfindung ist somit auch die Verwendung von nach Anspruch 1 bis 9 erhaltenen Polyätherpolyole als Ausgangsstoffe im Gemisch mit Polyisocyanaten zur Herstellung von Polyurethanen.

Als Ausgangskomponenten für die Herstellung von Polyurethanen kommen aliphatische, cycloaliphatische, araliphatische, aromatische und heterocyclische Polyisocyanate in Betracht, wie sie z.B. von W. Siefken in Justus Liebigs Annalen der Chemie 562, Seiten 75 bis 136, beschrieben werden, beispielsweise Äthylendiisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethylcyclohexan (DAS 1 202 785, Amerikanische Patentschrift 3 401 190), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4''-triisocyanat, Polyphenyl-polymethylen-polyisocyanate, wie sie durch Anilin-Formaldehyd-Kondensation und anschliessende Phosgenierung erhalten und z.B. in den britischen Patentschriften 874 430 und 848 671 beschrieben werden, m- und p-Isocyanatophenyl-sulfonyl-isocyanate gemäss der amerikanischen Patentschrift 3 454 606, perchlorierte Arylpolyisocyanate, wie sie z.B. in der deutschen Auslegeschrift 1 157 601 (amerikanische Patentschrift 3 277 138) beschrieben werden, Carbodiimidgruppen aufweisende Polyisocyanate, wie sie in der deutschen Patentschrift 1 092 007 (amerikanische Patentschrift 3 152 162) beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in der amerikanischen Patentschrift 3 001 973, in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der amerikanischen Patentschrift 3 394 164 beschrieben werden, acylierte Harnstoffgruppen aufweisende Polyisocyanate gemäss der deutschen Patentschrift 1 230 778, Biuretgruppen aufweisende Polyisocyanate, wie sie z.B. in der deutschen Patentschrift 1 101 394 (amerikanische Patentschriften 3 124 605 und 3 201 372) sowie in der britischen Patentschrift 889 050 beschrieben werden, durch Telomerisationsreaktionen hergestellte Polyisocyanate, wie sie z.B. in der amerikanischen Patentschrift 3 654 106 beschrieben werden, Estergruppen aufweisende Polyisocyanate, wie sie zum Beispiel in den britischen Patentschriften 965 474 und 1 072 956, in der amerikanischen Patentschrift 3 567 763 und in der deutschen Patentschrift 1 231 688 genannt werden, Umsetzungsprodukte der obengenannten Isocyanate mit Acetalen gemäss der deutschen Patentschrift 1 072 385 und polymere Fettsäurereste enthaltende Polyisocyanate gemäss der amerikanischen Patentschrift 3 455 883.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzten. Ferner ist es möglich, beliebige Mischungen der vorgenannten Polyisocyanate zu verwenden.

Besonders bevorzugt werden in der Regel die technisch leicht zugänglichen Polyisocyanate, z. B. das 2,4- und 2,6-Toluylendiisocyanat sowie beliebige Gemische dieser Isomeren («TDI»), Polyphenyl-polymethylen-polyisocyanate, wie sie durch Anilin-Formaldehyd-Kondensation und anschliessende Phosgenierung hergestellt werden («rohes MDI») und Carbodiimidgruppen, Urethangruppen, Allophanatgruppen, Isocyanuratgruppen, Harnstoffgruppen oder Biuretgruppen aufweisenden Polyisocyanate («modifizierte Polyisocyanate»).

Neben den erfindungsgemäss hergestellten Polyäthern gegebenenfalls einzusetzende Ausgangskomponenten sind ferner Verbindungen mit mindestens zwei gegenüber loscyanaten reaktionsfähigen Wasserstoffatomen von einem Molekulargewicht in der Regel von 400–10 000. Hierunter versteht man neben Aminogruppen, Thiolgruppen oder Carboxylgruppen aufweisenden Verbindungen vorzugsweise Polyhydroxylverbindungen, insbesondere zwei bis acht Hydroxylgruppen aufweisende Verbindungen, speziell solche vom Molekulargewicht 800 bis 10 000, vorzugsweise 1000 bis 6000, z. B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind.

Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

Als Beispiele hierfür seien genannt: Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester und Terephthalsäure-bis-glykolester. Als mehrwertige Alkohole kommen z. B. Äthylenglykol, Propylenglykol-(1,2) und -(1,3), Butylenglykol-(1,4) und -(2,3), Hexandiol-(1,6), Octandiol-(1,8), Neopentylglykol, Cyclohexandimethanol(1,4-Bis-hydroxymethylcyclohexan), 2-Methyl-1,3-propandiol, Glycerin, Trimethylpropan, Hexantriol-(1,2,6), Butantriol-(1,2,4), Trimethyloläthan, Pentaerythrit, Chinit, Mannit und Sorbit, Methylglykosid, ferner Diäthylenglykol, Triäthylenglykol, Tetraäthylenglykol, Polyäthylenglykole, Dipropylenglykol, Polypropylenglykole, Dibutylenglykol und Polybutylenglykole in Frage. Die Polyester können anteilig endständige Carboxylgruppen aufweisen. Auch Polyester aus Lactonen, z. B. ε-Caprolacton oder Hydroxycarbonsäuren, z. B. ω-Hydroxycapronsäure, sind einsetzbar.

Auch durch Vinylpolymerisate modifizierte Polyäther, wie sie z. B. durch Polymerisation von Styrol und Acrylnitril in Gegenwart von Polyäthern entstehen (amerikanische Patentschriften 3 383 351, 3 304 273, 3 523 093, 3 110 695, deutsche Patentschrift 1 152 536), sind geeignet, ebenso OH-Gruppen aufweisende Polybutadiene.

Unter den Polythioäthern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten um Polythiomischäther, Polythioätherester oder Polythioätheresteramide.

Als Polyacetale kommen z. B. die aus Glykolen, wie Diäthylenglykol, Triäthylenglykol, 4,4'-Dioxäthoxydiphenyldimethylmethan, Hexandiol und Formaldehyd herstellbaren Verbindungen in Frage. Auch durch Polymerisation cyclischer Acetale lassen sich erfindungsgemäss geeignete Polyacetale herstellen.

Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diäthylenglykol, Triäthylenglykol oder Tetraäthylenglykol mit Diarylcarbonaten, z. B. Diphenylcarbonat, oder Phosgen hergestellt werden können.

Zu den Polyesteramiden und Polyamiden zählen z. B. die aus mehrwertigen gesättigten und ungesättigten Carbonsäuren bzw. deren Anhydriden und mehrwertigen gesättigten und ungesättigten Aminoalkoholen, Diaminen, Polyaminen und ihren Mischungen gewonnen, vorwiegend linearen Kondensate.

Auch bereits Urethan- oder Harnstoffgruppen enthaltende Polyhydroxylverbindungen sowie gegebenenfalls modifizierte natürliche Polyole, wie Rizinusöl, Kohlenhydrate oder Stärke, sind verwendbar. Auch Anlagerungsprodukte von Alkylenoxiden an Phenol-Formaldehyd-Harze oder auch an Harnstoff-Formaldehydharze sind erfindungsgemäss einsetzbar.

Vertreter dieser erfindungsgemäss zu verwendenden Verbindungen sind z. B. in High Polymers, Vol. XVI, «Polyurethanes, Chemistry and Technology», verfasst von Saunders-Frisch, Interscience Publishers, New York, London, Band I, 1962, Seiten 32–42 und Seiten 44–54 und Band II, 1964, Seiten 5–6 und 198–199, sowie im Kunststoff-Handbuch, Band VII, Vieweg-Höchtlen, Carl-Hanser-Verlag, München, 1966, z. B. auf den Seiten 45–71, beschrieben.

Als erfindungsgemäss gegebenenfalls einzusetzende Ausgangskomponenten kommen auch Verbindungen mit mindestens zwei gegenüber Isocyanaten reaktionsfähigen Wasserstoffatomen von einem Molekulargewicht 32–400 in Frage. Auch in diesem Fall versteht man hierunter Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8 gegenüber Isocyanaten reaktionsfähige Wasserstoffatome auf, vorzugsweise 2 oder 3 reaktionsfähige Wasserstoffatome.

Als Beispiele für derartige Verbindungen seien genannt: Äthylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bishydroxymethyl-cyclohexan, 2-Methyl-1,3-propandiol, Glyzerin, Trimethylolpropan, Hexantriol-(1,2,6), Trimehtyloläthan, Pentacrythrit, Chinit, Mannit und Sorbit, Diäthylenglykol, Triäthylenglykol, Tetraäthylenglykol, Polyäthylenglykole mit einem Molekulargewicht bis 400, Dipropylenglykol, Polypropylenglykole mit einem Molekulargewicht bis 400, Dibutylenglykol, Polybutylenglykole mit einem Molekulargewicht bis 400, Dibutylenglykol, Polybutylenglykole mit einem Molekulargewicht bis 400, 4,4'-Dihydroxydiphenylpropan, Di-hydroxymethyl-hydrochinon, Äthanolamin, Diäthanolamin, Triäthanolamin, 3-Aminopropanol, Äthylendiamin, 1,3-Diaminopropan, 1-Mercapto-3-aminopropan, 4-Hydroxy- oder -Amino-phthalsäure, Bernsteinsäure, Adipinsäure, Hydrazin, N,N'-Dimethylhydrazin, 4,4'-Diaminodiphenylmethan, Toluylendiamin, Methylen-bis-chloranilin, Methylen-bis-anthranilsäureester Diaminobenzoesäureester und die isomeren Chlorphenylendiamine.

Auch in diesem Fall können Mischungen von verschiedenen Verbindungen mit mindestens zwei gegenüber Isocyanaten reaktionsfähigen Wasserstoffatomen mit einem Molekulargewicht von 32–400 verwendet werden.

Selbstverständlich ist es auch möglich, die erfindungsgemäss einzusetzenden Startergemische als Kettenverlängerungs- bzw. Vernetzungsmittel einzusetzen.

Erfindungsgemäss können jedoch auch Polyhydroxylverbindungen eingesetzt werden, in welchen hochmolekulare Polyaddukte bzw. Polykondensate in feindisperser oder gelöster Form enthalten sind. Derartige modifizierte Polyhydroxylverbindungen werden erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) direkt in situ in den oben genannten, Hydroxylgruppen aufweisenden Verbindungen ablaufen lässt. Derartige Verfahren sind beispielsweise in den Deutschen Auslegeschriften 1 168 075 und 1 260 142, sowie den Deutschen Offenlegungsschriften 2 324 134, 2 423 984, 2 512 385, 2 513 815, 2 550 796, 2 550 797, 2 550 833 und 2 550 862 beschrieben. Es ist aber auch möglich, gemäss US-Patent 3 869 413 bzw. Deutscher Offenlegungsschrift 2 550 860 eine fertige wässrige Polymerdispersion mit einer Polyhydroxylverbindung zu vermischen und anschliessend aus dem Gemisch das Wasser zu entfernen.

Bei der Verwendung von modifizierten Polyhydroxylverbindungen der oben genannten Art als Ausgangskomponente im Polyisocyanat-Polyadditionsverfahren entstehen in vielen Fällen Polyurethankunststoffe mit wesentlich verbesserten mechanischen Eigenschaften.

Bei der Herstellung von geschäumten Polyurethankunststoffen können Wasser und/oder leicht flüchtige organische Substanzen als Treibmittel mitverwendet werden. Als organische Treibmittel kommen z. B. Aceton, Äthylacetat, halogensubstituierte Alkane wie Methylenchlorid, Chloroform, Äthyliden-chlorid, Vinylidenchlorid, Monofluortrichlormethan, Chlordifluormethan, Dichlordifluormethan, ferner Butan, Hexan, Heptan oder Diäthyläther in Frage. Eine Treibwirkung kann auch durch Zusatz von bei Temperaturen über Raumtemperatur unter Abspaltung von Gasen, beispielsweise von Stickstoff, sich zersetzenden Verbindungen, z. B. Azoverbindungen wie Azoisobuttersäurenitril, erzielt werden. Weitere Beispiele für Treibmittel sowie Einzelheiten über die Verwendung von Treibmitteln sind im Kunststoff-Handbuch, Band VII, herausgegeben von Vieweg und Höchtlen, Carl-Hanser-Verlag, München 1966, z. B. auf den Seiten 108 und 109, 453 bis 455 und 507 bis 510 beschrieben.

Erfindungsgemäss werden ferner oft Katalysatoren mitverwendet. Als mitzuverwendende Katalysatoren kommen solche der an sich bekannten Art in Frage, z. B. tertiäre Amine, wie Triäthylamin, Tributylamin, N-Methyl-morpholin, N-Äthyl-morpholin, N-Cocomorpholin, N,N,N',N'-Tetramethyl-äthylendiamin, 1,4-Diaza-bicyclo-(2,2,2)-octan, N-Methyl-N'-dimethylaminoäthyl-piperazin, N,N-Dimethylbenzylamin, Bis-(N,N-di-äthylaminoäthyl)-adipat, N,N-Diäthylbenzylamin, Pentamethyldiäthylentriamin, N,N-Dimethylcyclohexylamin, N,N,N',N'-Tetramethyl-1,3-butandiamin, N,N-Dimethyl-β-phenyläthylamin, 1,2-Dimethylimidazol, 2-Methylimidazol. Als Katalysatoren kommen auch an sich bekannte Mannichbasen aus sekundären Aminen, wie Dimethylamin, und Aldehyden, vorzugsweise Formaldehyd, oder Ketonen wie Aceton, Methyläthylketon oder Cyclohexanon und Phenolen, wie Phenol, Nonylphenol oder Bisphenol in Frage.

Gegenüber Isocyanatgruppen aktive Wasserstoffatome aufweisende tertiäre Amine als Katalysatoren sind z. B. Triäthanolamin, Triisopropanolamin, N-Methyldiäthanolamin, N-Äthyl-diäthanolamin, N,N-Dimethyl-äthanolamin, sowie deren Umsetzungsprodukte mit Alkylenoxid, wie Propylenoxid und/oder Äthylenoxid.

Als Katalysatoren kommen ferner Silaamine mit Kohlenstoff-Sizilium-Bindungen, wie sie z. B. in der deutschen Patentschrift 1 229 290 (entsprechend der amerikanischen Patentschrift 3 620 984) beschrieben sind, in Frage, z. B. 2,2,4-Trimethyl-2-silamorpholin und 1,3-Diäthylaminomethyl-tetramethyl-disiloxan.

Als Katalysatoren kommen auch stickstoffhaltige Basen wie Tetraalkylammoniumhydroxide, ferner Alkalihydroxide wie Natriumhydroxid, alkaliphenolate wie Natriumphenolat oder Alkalialkoholate wie Natriummethylat in Betracht. Auch Hexahydrotriazine können als Katalysatoren eingesetzt werden.

Erfindungsgemäss können auch organische Metallverbindungen, insbesondere organische Zinnverbindungen, als Katalysatoren verwendet werden.

Als organische Zinnverbindungen kommen vorzugsweise Zinn(II)-salze von Carbonsäuren wie Zinn(II)-acetat, Zinn(II)-acetat, Zinn(II)-äthylhexoat und Zinn(II)-laurat und die Zinn(IV)-Verbindungen, z. B. Dibutylzinnoxid, Dibutylzinndichlorid, Dibutylzinndiacetat, Dibutylzinndilaurat, Dibutylzinnmaleat oder Dioctylzinndiacetat in Betracht. Selbstverständlich können alle obengenannten Katalysatoren als Gemische eingesetzt werden.

Weitere Vertreter von erfindungsgemäss zu verwendenden Katalysatoren sowie Einzelheiten über die Wirkunsweise der Katalysatoren sind im Kunststoff-Handbuch, Band VII, herausgegeben von Vieweg und Höchtlen, Carl-Hanser-Verlag, München 1966, z. B. auf den Seiten 96 bis 102 beschrieben.

Die Katalysatoren werden in der Regel in einer Menge zwischen etwa 0,001 und 10 Gew.-%, bezogen auf die Menge an Verbindungen mit mindestens zwei gegenüber Isocyanaten reaktionsfähigen Wasserstoffatomen von einem Molekulargewicht von 400 bis 10 000, eingesetzt.

Erfindungsgemäss können auch oberflächenaktive Zusatzstoffe, wie Emulgatoren und Schaumstabilisatoren, mitverwendet werden. Als Emulgatoren kommen z. B. die Natriumsalze von Ricinusölsulfonaten oder Salze von Fettsäuren mit Aminen wie ölsaures Diäthylamin oder stearinsaures Diäthanolamin in Frage. Auch Alkali- oder Ammoniumsalze von Sulfonsäuren wie etwa von Dodecylbenzolsulfonsäure oder Dinaphthylmethandisulfonsäure oder von Fettsäuren wie Ricinolsäure oder von polymeren Fettsäuren können als oberflächenaktive Zusatzstoffe mitverwendet werden.

Als Schaumstabilisatoren kommen vor allem Polyäthersiloxane, speziell wasserlösliche Vertreter, in Frage. Diese Verbindungen sind im allgemeinen so aufgebaut, dass ein Copolymerisat aus Äthylenoxid und Propylenoxid mit einem Polydimethylsiloxanrest verbunden ist. Derartige Schaumstabilisatoren sind z. B. in den amerikanischen Patentschriften 2 834 748, 2 917 480 und 3 629 308 beschrieben.

Erfindungsgemäss können ferner auch Reaktionsverzögerer, z. B. sauer reagierende Stoffe wie Salzsäure oder organische Säurehalogenide, ferner Zellregler der an sich bekannten Art wie Paraffine oder Fettalkohole oder Dimethylpolysiloxane sowie Pigmente oder Farbstoffe und Flammschutzmittel der an sich bekannten Art, z. B. Tris-chloräthylphosphat, Trikresylphosphat oder Ammoniumphosphat und -polyphosphat, ferner Stabilisatoren gegen Alterungs- und Witterungseinflüsse, Weichmacher und fungistatisch und bakteriostatisch wirkende Substanzen sowie Füllstoffe wie Bariumsulfat, Kieselgur, Russ oder Schlämmkreide mitverwendet werden.

Weitere Beispiele von gegebenenfalls erfindungsgemäss mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkunsweise dieser Zusatzmittel sind im Kunststoff-Handbuch, Band VII, herausgegeben von Vieweg und Höchtlen, Carl-Hanser-Verlag, München 1966, z. B. auf den Seiten 103 bis 113 beschrieben.

Die Reaktionskomponenten werden erfindungsgemäss nach dem an sich bekannten Einstufenverfahren, dem Prepolymerverfahren oder dem Semiprepolymerverfahren zur Umsetzung gebracht, wobei man sich oft maschineller Einrichtungen bedient, z. B. solcher, die in der amerikanischen Patentschrift 2 764 565 beschrieben werden. Einzelheiten über Verarbeitungseinrichtungen, die auch erfindungsgemäss in Frage kommen, werden im Kunststoff-Handbuch, Band VI, herausgegeben von Vieweg und Höchtlen, Carl-Hanser-Verlag, München 1966, z. B. auf den Seiten 121 bis 205 beschrieben.

Bei der Schaumstoffherstellung wird erfindungsgemäss die Verschäumung oft in Formen durchgeführt. Dabei wird das Reaktionsgemisch in eine Form eingetragen. Als Formmaterial kommt Metall, z. B. Aluminium, oder Kunststoff, z. B. Epoxidharz, in Frage. In der Form schäumt das schaumfähige Reaktionsgemisch auf und bildet den Formkörper. Die Formverschäumung kann dabei so durchgeführt werden, dass das Formteil an seiner Oberfläche Zellstruktur aufweist, es kann aber auch so durchgeführt werden, dass das Formteil eine kompakte Haut und einen zelligen Kern aufweist. Erfindungsgemäss kann man in diesem Zusammenhang so vorgehen, dass man in die Form so viel schäumfähiges Reaktionsgemisch einträgt, dass der gebildete Schaumstoff die Form gerade ausfüllt. Man kann aber auch so arbeiten, dass man mehr schäumfähiges Reaktionsgemisch in die Form einträgt, als zur Ausfüllung des Forminneren mit Schaumstoff notwendig ist. Im letztgenannten Fall wird somit

unter «overcharging» gearbeitet; eine derartige Verfahrensweise ist z.B. aus den amerikanischen Patentschriften 3 178 490 und 3 182 104 bekannt.

Bei der Formverschäumung werden vielfach an sich bekannte «äussere Trennmittel», wie Siliconöle, mitverwendet. Man kann aber auch sogenannte «innere Trennmittel», gegebenenfalls im Gemisch mit äusseren Trennmitteln, verwenden, wie sie z.B. aus den deutschen Offenlegungsschriften 2 121 670 und 2 307 589 bekanntgeworden sind.

Erfindungsgemäss lassen sich auch kalthärtende Schaumstoffe herstellen (vgl. britische Patentschrift 1 162 517, deutsche Offenlegungsschrift 2 153 086).

Selbstverständlich können aber auch Schaumstoffe durch Blockverschäumung oder nach dem an sich bekannten Doppeltransportbandverfahren hergestellt werden.

Zusammenfassend lässt sich feststellen, dass die erfindungsgemäss hergestellten Polyäther gegenüber den bekannten Polyäthern des Standes der Technik folgende wesentliche Vorteile haben:

Bei gleicher Funktionalität und vergleichbarer Hydroxylzahl weisen die erfindungsgemässen Polyäther gegenüber den Polyäthern des Standes der Technik, wie z.B. den Trimethylolpropanpolyäthern oder den Polyäthern auf Basis von Saccharose oder Saccharose/Polyol-Mischungen eine erniedrigte Viskosität auf. Diese Eigenschaft ermoglicht es, die Polyäther unter gunstigen Voraussetzungen zu Polyurethanschaumstoffen umzusetzen. Durch die niedrige Viskosität wird eine erhöhte Förderleistung zu den Mischköpfen der bekannten Verschäumungsmaschinen erzielt und eine vollständigere, schnellere Durchmischung mit der Isocyanatkomponente erzielt. Das Reaktionsgemisch kann daher in kürzerer Zeit aufgebracht werden, bzw. kann bei gleicher Zeit mehr Reaktionsgemisch aufgebracht und dieses gleichmassiger verteilt werden.

Die niedrige Viskosität der erfindungsgemassen Polyäther führt ausserdem zu einem verbesserten Fliessvermögen der Reaktionsansätze beim Verschäumungsvorgang. Dadurch wird eine gleichmässigere Rohdichteverteilung der resultierenden Polyurethanschaumstoffe erzielt, was wiederum eine höhere Druckfestigkeit bewirkt.

Bei gleicher Viskosität des Reaktionsgemisches kann ein höherfunktioneller Polyäther eingesetzt werden, was zu rascherem Aushärten des Schaumstoffs führt.

Auch die Herstellung der Polyäther ist technisch vereinfacht:

Die erfindungsgemäss einzusetztenden Startergemische sind auch ohne Zusatz von Wasser oder niedrigviskosen Polyolen viskose bis niedrigviskose Flüssigkeiten. Beispielsweise ist die Viskosität eines Gemisches mit einer mittleren Funktionalität von 4,6 bei 80° C 1.195 mPa.s, bei 100° C liegt die Viskosität bei 324 mPa.s Nebenreaktionen, die als Folge der schlechten Rührbarkeit der Reaktionsmischungen auftreten, wie Karamelisierung, Verkohlung oder die Bildung von inneren Äthern, werden vermieden. Ein wirtschaftlicher Vorteil gegenüber Formit-Polyäthern liegt darin, dass die Hydrierkosten für die Reduktion der Formose zu Formit entfallen.

Ferner kann das Formosegemisch gewünschtenfalls weitgehend frei von Diolen (Alkoxylierungsprodukte von Wasser) hergestellt werden, da Formose – im Unterschied zu Zuckern wie Glucose – weitgehend wasserfrei eingesetzt werden kann.

Die erfindungsgemässen Polyäther weisen ferner einen deutlich verringerten Anteil an reduzierenden Gruppen auf und sind stattdessen an den Carbonylfunktionen acetalisiert bzw. ketalisiert. Hierdurch treten keine Karamelisierungsreaktionen unter unangenehmer Geruchsbildung und keine Kernverfärbungen der Schaumstoffe auf. Diese erfindungsgemässen Polyäther sind auch klare, wenig gefärbte Verbindungen.

Erfindungsgemässe Polyäthergemische sind mit hoch- und niedermolekularen Polyhydroxylverbindungen, aber auch mit Isocyanaten besser verträglich als Rohformosen.

Ausserdem enthalten sie bei der Verwendung von Propylenoxid erhebliche Anteile an primären Hydroxylgruppen und sind somit wesentlich reaktiver als unter alkalischer Katalyse hergestellte Polyäther. Weiterhin zeigen die Verfahrensprodukte niedrige Säurezahlen (vgl. Beispiel 1).

Zur Durchführung der nachstehenden Ausführungsbeispiele diente ein Autoklav, der mit einer Heiz- und Kühlvorrichtung, einem Rührwerk, einer Einrichtung zum Verdrängen der Luft (z.B. Vakuumanschluss und Stickstoffzuleitung), einer Vorrichtung zur Azeotropentwässerung und einer Vorrichtung zur Dosierung des Alkylenoxids versehen war. Die folgenden Beispiele erläutern das erfindungsgemässe Verfahren. Wenn nicht anders vermerkt, sind Zahlenangaben als Gewichtsteile bzw. Gewichtsprozente zu verstehen.

Beispiel 1

A) Herstellung der Formose

Die Herstellung der Formose erfolgte genau nach den Angaben des Beispiels 1 der DOS 2 639 084 im Rahem eines verzehnfachten halbtechnischen Ansatzes, jedoch mit dem Unterschied, dass die Formose-Bildung bei einem Restgehalt von 1,7% Formaldehyd anstatt 1,3% Formaldehyd abgebrochen wurde und dass ferner die Blei- und Kaliumionen an einem sauren handelsüblichen Kationenaustauscher fixiert wurden, anstatt die Blei-II-Ionen mit Kaliumcarbonat zu fällen. Durch diese Massnahme wurde die Formose-Lösung vollständig von Blei- und Kaliumionen befreit und damit eine Teilentsalzung durchgeführt.

Man erhält einen 4%Wasser enthaltenden Formosesirup mit einer Viskosität von 114 000 mPas bei 35° C mit folgender molekularer Verteilung:

Verbindungen mit 2 C-Atomen: 3,3%
Verbindungen mit 3 C-Atomen: 7,4%
Verbindungen mit 4 C-Atomen: 16,5%
Verbindungen mit 5 C-Atomen: 36,0%
Verbindungen mit 6 C-Atomen: 27,0%
Verbindungen mit 7 C-Atomen: 8,6%

Verbindungen mit 8 C-Atomen: 1,2%

Das mittlere Molekulargewicht dieser Formose beträgt 158, die mittlere Hydroxylfunktionalität ist 4,14. Der Gehalt an reduzierenden Verbindungen, berechnet als Glukose, beträgt 71%.

B) Erfindungsgemässes Verfahren

Vier Ansätze aus jeweils 100 g des Formosesirups werden unter Stickstoffatmosphäre mit je 0,5 g eines Komplexes aus 1 Mol Bortrifluorid und 1 Mol Essigsäure bei Raumtemperatur unter Rühren vermischt. Die vier Ansätze werden unter Stickstoffatmosphäre gut gerührt und die folgenden Mengen an Propylenoxid bei 49° C im Verlauf von 2 Stunden gleichmässig und langsam zugetropft:

a) 58 g Propylenoxid (1 Mol)
b) 87 g Propylenoxid (1,5 Mol)
c) 116 g Propylenoxid (2 Mol)
d) 232 g Propylenoxid (4 Mol)

Nachdem mit Natronlauge oder wässriger 25%iger Ammoniaklösung ein pH-Wert von 7,3 eingestellt wurde, werden die Ansätze a) bis d) bei 50° C im Vakuum von Spuren an Propylenoxid und kleinen Mengen an Wasser befreit. Man erhält Formose-Polyäther mit überraschend niedriger Viskosität und geringem Anteil an reduzierend wirkenden Gruppen.

a) Ausbeute: 155 g; OH-Zahl: 880; Säurezahl: 0,7; Viskosität: 19 000 mPas/35° C; reduzierend wirkender Anteil (berechnet als Glukose-Polyäther): 19,8%.

Der geringe Gehalt an reduzierend wirkenden Zuckeranteilen zeigt, dass bei der Propoxylierung überraschenderweise die Carbonylgruppen in der Formose in erheblichem Umfang acetalisiert bzw. ketalisiert wurden, vermutlich über die ringöffnende Addition von Propylenoxid zu 1,3-Dioxolanderivaten als Primärschritt.

b) Ausbeute: 183 g; OH-Zahl: 730; Säurezahl: 0,6; Viskosität: 16 840 mPas/35° C; Anteil an reduzierenden Verbindunen: 14,6%.

c) Ausbeute: 207 g; OH-Zahl: 640; Säurezahl: 0,6; Viskosität: 5 600 mPas/35° C; Anteil an reduzierenden Verbindungen: 12,2%.

d) Ausbeute: 315 g; OH-Zahl: 420; Säurezahl: 0,6; Viskosität: 3 890 mPas/35° C; Anteil an reduzierend wirkenden Verbindungen: 3,5%.

Wie eine einfache Rechnung ergibt, sind beim erfindungsgemässen Verfahren in Ansatz a) ca. 56%, bei b) ca. 63%, bei c) ca. 64% und in Ansatz d) ca. 80% der in der Formose vorhandenen Aldehyd- bzw. Ketogruppen acetalisiert bzw. ketalisiert worden. In Anbetracht der hohen Konzentration an freien OH-Gruppen und der 4 Gew.-% Wasser im Startergemisch, welche mit den Carbonylgruppen hinsichtlich der Reaktion mit Propylenoxid konkurrieren, ist dies ein ausserordentlich überraschender Befund.

Obwohl im vorliegenden Beispiel eine nur teilentsalzte Formose eingesetzt wurde, d.h. eine Formose, aus welcher lediglich die Metallkationen entfernt wurden, während die durch Cannizzaro-Reaktion und gekreuzte Cannizzaro-Reaktion entstandenen Anionen (Ameinsensäure, Milchsäure und verschiedene Zuckersäuren) im Reaktionsgemisch belassen wurden, zeigen die niedrigen Säurezahlen der Verfahrensprodukte, dass der Hauptteil der vorhandenen Säuren in Polyätherester mit endständigen Hydroxylgruppen übergeführt wurde.

Alle Polyäthergemische a) bis d) sind mit hoch- und niedermolekularen Polyhydroxylverbindungen, aber auch mit Isocyanaten besser verträglich als entwässerte Rohformosen. Von besonderer Bedeutung ist ferner, dass die gemäss a) bis d) erhaltenen Polyäther bei ihrer Reaktion mit Polyisocyanaten wesentlich aktiver sind als üblicherweise durch $OH^{\ominus}$-Katalyse in Gegenwart von Natronlauge oder Kalilauge hergestellte Formose-Polyäther. Die Lewis-Säure-katalysierte Polyaddition des Propylenoxids an Formose läuft offenbar ziemlich selektiv nach folgendem Schema ab:

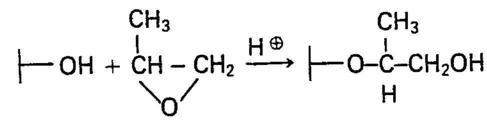


führt also zu primären Hydroxylgruppen, während die mit Basen katalysierte Polyaddition statistisch erfolgt und daher mindestens 50% an sekundären Hydroxylgruppen im Polyäther vorliegen. Darüberhinaus führt die $OH^{\ominus}$-katalysierte Reaktion zu dunkelgefärbten Produkten und ist von Zersetzungsreaktionen der Formosen begleitet.

Aus den Polyäthergemischen a) bis d) lassen sich nach bekannten Rezepturen harte bis halbharte Polyurethanschaumstoffe herstellen.

Bei der Polyaddition von etwa 2500 g Propylenoxid analog zu d) an 100 g Formose in Gegenwart erhöhter Mengen an $BF_3$-Acetat (ca. 5,5 g) werden Polyäther im OH-Zahl-Bereich von 56 bis 60 erhalten, die für die Herstellung von Weichschaum gut geeignet sind.

Beispiel 2

Beispiel 1 wird wiederholt, jedoch unter Verwendung einer Formose, die analog zu Beispiel 1A hergestellt wurde, wobei jedoch die Kondensation des Formaldehyds bei einem Restformaldehydgehalt von 1,3% abgebrochen wurde. Die von Kationen befreite Formose, welche noch einen Gehalt von ca. 1,5 Gew.-% an Ameinsensäure, Milchsäure und Zuckersäuren aufweist, besitzt folgende molekulare Verteilung:

Verbindungen mit 2 C-Atomen: 0,3%
Verbindungen mit 3 C-Atomen: 2,1%
Verbindungen mit 4 C-Atomen: 4,4%
Verbindungen mit 5 C-Atomen: 25,2%
Verbindungen mit 6 C-Atomen: 45,1%
Verbindungen mit 7 und mehr C-Atomen: 22,9%.

Das mittlere Molekulargewicht der Formose beträgt 167, 5, die mittlere Hydroxylfunktionalität 4,79, die OH-Zahl 1524 und der Anteil an reduzierend wirkenden Zuckern (berechnet als Glukose) 70%.

a) Bei der Umsetzung des ca. 3,8% Wasser enthaltenden Formosesirups gemäss Beispiel 1 B, Variante d), erhält man einen Formose-Polyäther,

der einen reduzierend wirkenden Anteil (berechnet als Glukose-Polyäther) von nur noch 8,2%, eine OH-Zahl von 425 und eine Säurezahl von 0,8 aufweist. Die Viskosität des Polyäthers beträgt 5500 mPas/35° C.

b) In ein Gemisch aus 100g des obigen Formosesirups (Wassergehalt 3,8%) und 2g des Komplexes aus 1 Mol Bortrifluorid und 1 Mol Essigsäure werden unter Stickstoff insgesamt 2350g Propylenoxid unter Rühren eingetropft. Nach Zusatz von jeweils 235g Propylenoxid werden dabei in das Reaktionsgemisch nochmals jeweils 0,5g des Katalysators eindosiert. Man erhält einen Polyäther mit einer OH-Zahl von 60, welcher einen reduzierenden Anteil von nur noch 0,4% enthält.

Variante a) des vorliegenden Beispiels wird wiederholt, jedoch unter Verwendung der folgenden Katalysatoren anstelle von 0,5g Bortrifluorid-acetat:

a 1) 0,8g eines Addukts aus 1 Mol Bortrifluorid und 1 Mol Essigsäureanhydrid;

a 2) 0,9g eines Addukts aus 1 Mol Bortrifluorid und 1 Mol des gemischten Anhydrids aus Essigsäure und Capronsäure;

a 3) 1,2g eines Addukts aus 1 Mol Bortrifluorid und 1 Mol des gemischten Anhydrids aus Benzoesäure und Essigsäure;

a 4) 1,8g des Additionsprodukts aus 1 Mol Bortrifluorid und 1 Mol des gemischten Anhydrids aus Essigsäure und Ölsäure;

a 5) 0,85g des Additionsproduktes aus 1 Mol Bortrifluorid und 1 Mol Maleinsäureanhydrid, gelöst in 3g Essigsäure;

a 6) 3,4g eines Addukts aus 1 Mol Bortrifluorid und 1 Mol Hexahydrophthalsäureanhydrid;

a 7) 2,5g eines Addukts aus 1 Mol Trimellitsäureanhydrid und 2 Mol Bortrifluorid, gelöst in 4g Essigsäure.

Die Polyaddition des Propylenoxids an das Startergemisch verläuft in allen Fällen etwa gleich schnell wie in Variante a); die Ausbeute beträgt ca. 95 bis 98% (der Ausbeuteverlust von ca. 2 bis 5% Propylenoxid ist darauf zurückzuführen, dass ein kleiner Teil des Propylenoxids durch den Inertgasstrom mitgerissen wird).

Nach der Aufarbeitung der gemäss a) und b) bzw. a 1) bis a 7) erhaltenen Produkte kann neuerlich Katalysator zugesetzt werden, worauf man unter einem Überdruck von 0,4 bar im Autoklaven bei 55° C durch Zudosierung von Äthylenoxid an die Propylenglykolsegmente der Polyäther noch Polyäthylenglykolsegmente anknüpfen kann. Ebenso ist es möglich, in dieser zweiten Stufe andere cyclische Äther, beispielsweise Epichlorhydrin, Styroloxid, Cyclohexanoxid oder Vinyloxiran an das Polypropylenoxid anzulagern.

Beispiel 3

Herstellung der Formose:

Die Herstellung der Formose erfolgt nach Beispiel 2 der DOS 2 639 084. Die Formosebildung wird wie in diesem Beispiel beschrieben bei einem Restgehalt von 8% Formaldehyd abgebrochen. Anschliessend wird die Formose analog zu Beispiel 1A von Kationen befreit und eingeengt.

Man erhält einen ca. 3% Wasser enthaltenden Formosesirup mit einer Viskosität von 12 500 mPa.s bei 35° C, einem mittleren Molekulargewicht von 104, einer mittleren Hydroxylfunktionalität von 2,39, einer OH-Zahl von 1260 und einem Zuckergehalt von 75%, berechnet als Glukose. Die Formose hat folgende molekulare Zusammensetzung:

Verbindungen mit 2 C-Atomen: 16,8%
Verbindungen mit 3 C-Atomen: 21,0%
Verbindungen mit 4 C-Atomen: 29,9%
Verbindungen mit 5 C-Atomen: 25,1%
Verbindungen mit 6 C-Atomen: 7,2%

100g des Formosesirups werden gemäss Beispiel 1B mit 116g Propylenoxid umgesetzt. Man erhält einen Formose-Polyäther, der einen Zuckeranteil von nur noch 13,4% (berechnet als Glukose-Polyäther) aufweist. Die Viskosität des Polyäthers bei 35° C beträgt 2400 mPa.s, die OH-Zahl 380 und die Säurezahl 0,7.

Bei einem höheren Wassergehalt des Startergemisches liefern nur die erfindungsgemäss besonders bevorzugten Komplexe aus Bortrifluorid und Carbonsäuren bzw. Carbonsäureanhydrid befriedigende Ergebnisse, während andere saure Katalysatoren nur geringe Propoxylierungsausbeuten ergeben. Infolge der im Reaktionsgemisch vorhandenen relativ grossen Wassermengen ist der Anteil der acetalisierten bzw. ketalisierten Carbonylgruppen in den Formosepolyäthern sehr gering (zwischen ca. 8 und 14% der in der Formose vorhandenen Reduktionsäquivalente).

Beispiel 4

200 Teile einer 50%igen wässrigen Lösung der in Beispiel 2 verwendeten Formose werden mit 287 Teilen einer 30%igen Formaldehydlösung vermischt und das Gemisch im Wasserstrahlvakuum auf einen Wassergehalt von 3,5% eingeengt. Die so erhaltene halbacetalisierte Formose wird unter völligem Luftausschluss mit 1,2 Teilen Bortrifluoridacetat versetzt und analog zu Beispiel 1 mit 196 Teilen Propylenoxid alkoxyliert. Man erhält 370 Teile eines Acetalsegmente und Polyacetalsegmente enthaltenden Formosepolyäthers mit einer OH-Zahl von ca. 405 und einer Säurezahl von 1,4. Es ist besonders überraschend, dass das Verfahrensprodukt nur ca. 4% des cyclischen 1,3-Dioxolans der Formel

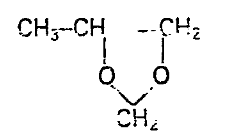

als Nebenprodukt enthält.

Beispiel 5

Das Beispiel beschreibt die Verwendung von Mischungen aus Formose und natürlichen Mono- bzw. Disacchariden sowie Invertzuckern als Startergemisch für das erfindungsgemässe Verfahren.

Jeweils 100g einer 50%igen wässrigen Lösung der in Beispiel 2 verwendeten Formose werden

mit je 50g der folgenden Saccharide vermischt und die erhaltene klare Lösung im Wasserstrahlvakuum auf einen Wassergehalt von 3,5% eingeengt:

a) 50g Rohrzucker
b) 50g D-Glukose
c) 50g Milchzucker
d) 50g Blütenbienenhonig
e) 50g eines durch enzymatischen Abbau von Maisstärke hergestellten Zuckergemisches
f) 50g eines künstlichen Invertzuckers, der durch Spaltung von Rohrzucker an einem sauren Ionenaustauscher hergestellt wurde.

Die Mischungen a) bis f) werden analog zu Beispiel 1B mit 232g Propylenoxid in Gegenwart von 0,8g des Additionsproduktes aus 1 Mol Bortrifluorid und 1 Mol Essigsäureanhydrid unter sorgfältigem Luftausschluss in eine Stickstoffatmosphäre alkoxyliert. Man erhält Polyätherpolyole mit folgenden Ausbeuten und Hydroxylzahlen:

a) 318g; OH-Zahl: 460
b) 319g; OH-Zahl: 463
c) 315g; OH-Zahl: 480
d) 309g; OH-Zahl: 489
e) 305g; OH-Zahl: 485
f) 310g; OH-Zahl: 479.

Die so erhaltenen gelblichen bis honigfarbenen Polyätherpolyole besitzen Säurezahlen zwischen 0,6 und 0,9.

Beispiel 6
Herstellung der Formose

Die Formose wurde wie folgt unter Verwendung von Calciumhydroxid als Katalysator und der in Beispiel 1A beschriebenen Formose als Co-Katalysator hergestellt:

In 2860g einer 37%igen wässrigen Formaldehydlösung werden 81g eines etwa 90%igen Formosesirups gelöst, der als Cokatalysator dient und gemäss Beispiel 1A hergestellt wurde. Die Lösung wird nun auf 90° C erhitzt und es werden im Verlaufe von 4,5 Stunden gleichmässig und in kleinen Portionen 70g Calciumhydroxyd zudosiert, (pH = 8,8 bis 8,3). Anschliessend wird die erkaltete Lösung zunächst an einem handelsüblichen sauren Ionenaustauscher von Calcium-Ionen und dann an einem basischen Ionenaustauscher von Ameisensäure, Milchsäure und Zuckersäuren befreit und im Rotationsverdampfer bei 16 Torr und 58° C auf einen Wassergehalt von 3,5% eingeengt. Ausbeute: 1005g einer ca.3,5% Wasser enthaltenden gelben Formose.

Infolge der Verwendung von Calziumhydroxid als Katalysator für die Formaldehydkondensation wird ein grosser Anteil der Carbonylgruppen durch gekreuzte Cannizzaro-Reaktion zu Hydroxylgruppen reduziert, so dass der Zuckeranteil des Reaktionsgemisches nur 37,2%, berechnet als Glukose, beträgt. Die so erhaltene Formose hat ein mittleres Molekulargewicht von 146, eine mittlere Hydroxylfunktionalität von 4,06 und eine OH-Zahl von 1490. Sie besitzt folgende molekulare Verteilung:

Verbindungen mit 2 C-Atomen: 1,17%
Verbindungen mit 3 C-Atomen: 2,21%
Verbindungen mit 4 C-Atomen: 10,09%
Verbindungen mit 5 C-Atomen: 8,05%
Verbindungen mit 6 C-Atomen: 28,27%
Verbindungen mit 7 und mehr C-Atomen: 50,21%.

Erfindungsgemässes Verfahren:

100g der vollentsalzten, 3,5l Wasser enthaltenden Formose werden analog zu Beispiel 1B mit 232g Propylenoxid in Gegenwart von 0,8 Bortrifluoridacetat bei 58 bis 60° C alkoxyliert. Man erhält 327g eines Polyäthers mit einer OH-Zahl von 465 und einer Säurezahl von 0,7.

Die gemäss Beispiel 1 bis 6 hergestellten Polyätherpolyole eignen sich hervorragend für die Herstellung von Polyurethanschaumstoffen, insbesondere von Hartschaumstoffen. Infolge ihres geringen Gehaltes an freien Aldehyd- und Ketogruppen gehen die erfindungsgemässen Verfahrensprodukte während der Verschäumungsreaktion vorteilhafterweise keine Karamelisierungsreaktionen ein, welche zu einer unangenehmen Geruchsentwicklung führen würden; die aus ihnen hergestellten Polyurethanschaumstoffe weisen auch keine Kernverfärbung auf.

Beispiel 7

89g einer Polyolformulierung, hergestellt aus 95 Teilen des Formose-Polyäthers mit der OH-Zahl 425 gemäss Beispiel 2a, 5 Teilen eines auf Äthylamin gestarteten Polyäthylenoxids mit einer OH-Zahl von 490, 2,1 Teilen Wasser, 1,5 Teilen eines handeslüblichen Schaumstabilisators auf Basis eines Siloxan-Oxyalkylen-Mischpolymeren und 2,1 Teilen Dimethylcyclohexylamin werden mit 38g Dichlordifluormethan und 129g eines technischen Diphenylmethandiisocyanats mit einem Isocyanatgehalt von 31% mittels eines hochtourigen Rührers gut vermischt.

Man erhält einen harten, gelben, geschlossenzelligen Polyurethanschaum mit einem Raumgewicht von 27kg/m$^3$.

Beispiel 8

In ein Gemisch aus 100g des Formose-Polyäthers mit einer OH-Zahl von 60 gemass Beispiel 2b, 4g Wasser, 1,5 Teilen eines handelsüblichen Schaumstabilisators auf Basis eines Siloxan-Oxialkylen-Mischpolymeren, 0,25 Teilen Triäthylendiamin und 0,4 Teilen des Zinn-(II)-salzes der 2-Äthylcapronsäure werden 54g Toluylendiisocyanat (80% 2,4- und 20% 2,6-Isomeres) mit einem hochtourigen Rührer eingerührt. Nach einer Startzeit von ca. 10 Sekunden beginnt die Verschäumungsreaktion und es entsteht ein weisser, weicher, offenporiger, elastischer Polyurethanschaumstoff mit guten physikalischen Eigenschaften und einem Raumgewicht von etwa 26 kg/m$^3$.

**Patentansprüche**

1) Verfahren zur Herstellung von Polyätherpolyolen mit einem durchschnittlichen Molekulargewicht im Bereich von 200 bis 10 000 und mit einer durchschnittlichen Hydroxylfunktionalität von 2,0 bis 7,0 durch Anlagerung eines oder mehrerer cyclischer Äther, in Gegenwart eines sauren Katalysators, an einen Starter, bestehend aus Zuckern und gegebenenfalls mehrwertigen Alkoholen, dadurch gekennzeichnet, dass als Starter

A) Formose, welche gegebenenfalls in α-Stellung aldolisiert ist, oder

B) flüssige Gemische aus

a) hoch- und/oder niedermolekularen Polyhydroxylverbindungen und/oder Mono- oder Disacchariden und/oder natürlichen oder künstlichen Invertzuckern und

b) Formose, welche gegebenenfalls in α-Stellung aldolisiert ist,

eingesetzt werden, wobei Formosen ausgenommen sind, welche direkt aus formaldehydhaltigen Synthesegasen hergestellt worden sind.

2) Verfahren nach Anspruch 1, dadurch gekennzeichnet, dass als Starter 20 bis 80gew.-%ige Lösungen von kristallinen Mono- bzw. Disacchariden und/oder natürlichen bzw. künstlichen Invertzuckern in Formose eingesetzt werden.

3) Verfahren nach Anspruch 1, dadurch gekennzeichnet, dass als Starter 30 bis 70 gew.-%ige Lösungen von kristallinen Mono- bzw. Disacchariden und/oder natürlichen bzw. künstlichen Invertzuckern in Formose eingesetzt werden.

4) Verfahren nach Ansprüchen 1 bis 3, dadurch gekennzeichnet, dass der Starter 2 bis 100 Gew.-%, bezogen auf Formose, an zwei- oder mehrwertigen Alkoholen mit einem Molekulargewicht zwischen 62 und 300 und/oder Polyäther-, Polyester-, Polyacetal- und/oder Polycarbonatpolyole mit einem Molekulargewicht zwischen 300 und 4000 enthält.

5) Verfahren nach Ansprüchen 1 bis 4, dadurch gekennzeichnet, dass Formosen mit einem Molekulargewicht zwischen 92 und 360 eingesetzt werden.

6) Verfahren nach Ansprüchen 1 bis 5, dadurch gekennzeichnet, dass Formosen mit einem Gehalt an reduzierenden Verbindungen, berechnet als Glukose, von 4 bis 85 Gew.-% eingesetzt werden.

7) Verfahren nach Ansprüchen 1 bis 6, dadurch gekennzeichnet, dass als cyclische Äther Äthylenoxid und/oder Propylenoxid eingesetzt werden.

8) Verfahren nach Ansprüchen 1 bis 7, dadurch gekennzeichnet, dass als saurer Katalysator ein Addukt einer Carbonsäure oder eines Carbonsäureanhydrids an Bortrifluorid eingesetzt wird.

9) Verfahren nach Ansprüchen 1 bis 8, dadruch gekennzeichnet, dass der Starter 0,5 bis 4 Gew.-% Wasser enthält.

10) Verwendung von nach Ansprüchen 1 bis 9 erhaltenen Polyätherpolyolen im Gemisch mit Polyisocyanaten zur Herstellung von Polyurethanen.

**Claims**

1. Process for the production of polyether polyols having an average molecular weight in the range of 200 to 10,000 and an average hydroxyl functionality of 2.0 to 7.0 by the addition of one or more cyclic ethers, in the presence of an acid catalyst, to a starter consisting of sugars and optionally polyhydric alcohols, characterised in that as starters

A) formose which is optionally aldolated in the α-position, or

B) liquid mixtures of

a) high and/or low molecular weight polyhydroxyl compounds and/or mono- or di-saccharides and/or natural or synthetic invert sugars and

b) formose which is optionally aldolated in the α-position,

are employed, those formoses being excluded which have been directly produced from formaldehyde-containing synthesis gases.

2. Process according to Claim 1, characterised in that 20 to 80% by weight solutions of crystalline mono- or disaccharides and/or natural or synthetic invert sugars in formose are used as starters.

3. Process according to Claim 1, characterised in that 30 to 70% by weight solutions of crystalline mono- or di-saccharides and/or natural or synthetic invert sugars in formose are used as starters.

4. Process according to Claims 1 to 3, characterised in that the starter contains 2 to 100% by weight, based on formose, of di- or poly-hydric alcohols having a molecular weight between 62 and 300 and/or polyether, polyester, polyacetal and/or polycarbonate polyols having a molecular weight between 300 and 4,000.

5. Process according to Claims 1 to 4, characterised in that formoses having a molecular weight between 92 and 360 are used.

6. Process according to Claims 1 to 5, characterised in that formosed having a content of reducing compounds, expressed as glucose, of 4 to 85% by weight are used.

7. Process according to Claims 1 to 6, characterised in that ethylene oxide and/or propylene oxide are used as cyclic ethers.

8. Process according to Claims 1 to 7, characterised in that an adduct of a carboxylic acid or of a carboxylic acid anhydride with boron trifluoride is used as acid catalyst.

9. Process according to Claims 1 to 8, characterised in that the starter contains 0.5 to 4% by weight of water.

10. Use of polyether polyols obtained according to Claims 1 to 9 in mixture with polyisocyanates for the production of polyurethanes.

**Revendications**

1. Procédé pour la préparation de polyétherpolyols ayant un poids moléculaire moyen dans l'intervalle de 200 à 10 000 et une fonctionalité moyenne en hydroxyle de 2,0 à 7,0 par addition d'un ou plusieurs éthers cycliques, en présence d'un catalyseur acide, sur un composé d'amorçage consistant en sucres et éventuellement en

polyalcools, caractérisé en ce que l'on utilise comme composés d'amorçage

A) un formose qui est éventuellement aldolisé en position α, ou

B) des mélanges liquides de

a) composés polyhydroxylés de haut poids moléculaire et/ou de bas poids moléculaire et/ou mono- ou di-saccharides et/ou sucres invertis et de

b) formose qui est éventuellement aldolisé en position α,

à l'exception des formoses qui sont produits directement à partir de gaz de synthèse contenant du fomaldéhyde.

2. Procédé selon la revendication 1, caractérisé en ce que l'on utilise comme composés d'amorçage des solutions à 20–80% en poids de mono- ou di-saccharides cristallisés et/ou de sucres invertis naturels ou synthétiques dans le formose.

3. Prodédé selon la revendication 1, caractérisé en ce que l'on utilise comme composés d'amorçage des solutions à 30–70% en poids de mono- ou di-saccharides cristallisés et/ou de sucres invertis naturels ou synthétiques dans le formose.

4. Procédé selon les revendications 1 à 3, caractérisé en ce que le composé d'amorçage contient 2 à 100% en poids, par rapport au formose, de di- ou poly-alcools d'un poids moléculaire compris entre 62 et 300 et/ou de polyéther-, polyester- polyacétal- et/ou polycarbonates-polyols d'un poids moléculaire compris entre 300 et 4000.

5. Procédé selon les revendications 1 à 4, caractérisé en ce que l'on utilise des formoses d'un poids moléculaire compris entre 92 et 360.

6. Procédé selon les revendications 1 à 5, caractérisé en ce que l'on utilise des formoses ayant une teneur en composés réducteurs, calculée en glucose, de 4 à 85% en poids.

7. Procédé selon les revendications 1 à 6, caractérisé en ce que l'on utilise comme éthers cycliques l'oxyde d'éthylène et/ou l'oxyde de propylène.

8. Procédé selon les revendications 1 à 7, caractérisé en ce que l'on utilise comme catalyseur acide un produit d'addition d'un acide carboxylique ou d'un anhydride d'acide carboxylique sur le trifluorure de bore.

9. Procédé selon les revendications 1 à 8, caractérisé en ce que le composé d'amorçage contient 0,5 à 4% en poids d'eau.

10. Utilisation de polyétherpolyols obtenus selon les revendications 1 à 9 en mélange avec des polyisocyanates pour la préparation de polyuréthanes.